# EUROPEAN PATENT APPLICATION

(11) **EP 2 253 271 A1**
(43) Date of publication of application: **24.11.2010**
(21) Application number: 09722593.2
(22) Date of filing: 21.01.2009
(51) Int. Cl.: A61B 5/157, A61B 5/1473, A61B 5/151

(54) **CIRCUIT BOARD FOR COLLECTING BODY FLUID AND BIOSENSOR**

(30) Priority: 21.03.2008 JP 2008073541
(71) Applicant: Nitto Denko Corporation, Ibaraki-shi Osaka 567-8680 (JP)
(72) Inventor: NAITO, Toshiki, Ibaraki-shi Osaka 567-8680 (JP); OHSAWA, Tetsuya, Ibaraki-shi Osaka 567-8680 (JP)
(74) Representative: HOFFMANN EITLE
(86) International application number: PCT/JP2009/050820
(87) International publication number: WO 2009/116313

(57) **Abstract**

A circuit board for body fluid collection include a first substrate including a puncture needle; a second substrate disposed at an upstream side of the first substrate in a direction of puncturing with the puncture needle with a space provided therebetween; a flexible insulation layer that extends between the first substrate and the second substrate; and a conductive pattern provided on the flexible insulation layer, the conductive pattern integrally including an electrode, a terminal, and a wiring that electrically connects the electrode and the terminal.

## Description

### TECHNICAL FIELD

The present invention relates to a circuit board for body fluid collection, and a biosensor. In particular, the present invention relates to a circuit board for body fluid collection, and a biosensor including the same.

### BACKGROUND ART

A biosensor that measures, for example, a blood-sugar level by causing bleeding by puncturing with a puncture needle, and by allowing the blood to react with a reagent that is applied on an electrode has been known.
For example, a biosensor system has been proposed in which a puncture needle and a biosensor chip are driven simultaneously by a driving unit to puncture with a puncture needle, and a sample is collected with the biosensor chip; and the biosensor chip and a measurement device that analyzes and measures the sample based on information from the biosensor chip are connected by deformable wirings (for example, see Patent Document 1 below).
Patent Document 1
Japanese Unexamined Patent Publication No. 2007-282864

### DISCLOSURE OF THE INVENTION

### PROBLEM TO BE SOLVED BY THE INVENTION

In the biosensor system described in abovementioned Patent Document 1, the puncture needle and the biosensor chip are separately provided, and further, the biosensor chip and the measurement device are connected by deformable wirings. Therefore, there are disadvantages in that assembling its components is troublesome, and the device increases in its size.
An object of the present invention is to provide a circuit board for body fluid collection that can reduce assembling procedures of components, reduce the size of the device, and further, improve measurement reliability; and a biosensor including the circuit board for body fluid collection.

### MEANS FOR SOLVING THE PROBLEM

To achieve the above-described object, a circuit board for body fluid collection of the present invention includes a first substrate having a puncture needle, a second substrate that is disposed at an upstream side of the first substrate in a direction of puncturing with the puncture needle with a space provided therebetween, a flexible insulation layer that extends between the first substrate and the second substrate, and a conductive pattern provided on the flexible insulation layer, the conductive pattern integrally including an electrode, a terminal, and a wiring that electrically connects the electrode and the terminal.

In this circuit board for body fluid collection, the conductive pattern integrally including the electrode, the terminal, and the wiring is provided on the flexible insulation layer that extends between the first substrate having the puncture needle and the second substrate. Therefore, assembling procedures of components can be reduced. Also, the size of the device can be reduced. Furthermore, the wiring is provided on the flexible insulation layer as the conductive pattern. Therefore, occurrence of disconnection can be reduced, and improvement in measurement reliability can be achieved.

It is preferable that, in the circuit board for body fluid collection of the present invention, the terminal is disposed so as to overlap with the second substrate when projected in the thickness direction.
When the terminal is disposed so as to overlap with the second substrate, the terminal can be supported reliably. Therefore, connection reliability between the terminal and the external measurement device electrically connected thereto can be improved, and improvement in measurement reliability can be achieved.

It is preferable that, in the circuit board for body fluid collection of the present invention, the electrode is disposed so as to overlap with the first substrate when projected in the thickness direction.
When the electrode is disposed so as to overlap with the first substrate, the electrode can be disposed in the proximity of the puncture needle. Therefore, the body fluid that was caused to flow out by the puncturing with the puncture needle can be brought easily in contact with the electrode. As a result, a measurement can be easily performed on a body fluid component.

It is also preferable that, in the circuit board for body fluid collection of the present invention, the electrode is disposed so as to overlap with a portion between the first substrate and the second substrate when projected in the thickness direction.
When the electrode is disposed so as to overlap with a portion between the first substrate and the second substrate, by bending of the flexible insulation layer, the puncture needle and the electrode can be brought into contact with each other. Therefore, the body fluid adhered to the puncture needle after the puncturing can be brought into contact with the electrode. As a result, a measurement can be easily performed on a body fluid component.

It is preferable that, in the circuit board for body fluid collection of the present invention, the electrode is disposed so as to overlap with the second substrate when projected in the thickness direction.
When the electrode is disposed so as to overlap with the second substrate, the electrode can be supported reliably. Therefore, the body fluid that was caused to flow out by puncturing with the puncture needle can be brought into contact with the electrode reliably. As a result, a reliable measurement can be easily performed on a body fluid component.

A biosensor of the present invention includes a housing and the above-described circuit board for body fluid collection, wherein the circuit board for body fluid collection is accommodated in the housing, the second substrate is fixed, and the first substrate is supported so as to be capable of advancing from inside the housing to outside the housing.
With this biosensor, the first substrate having the puncture needle can be advanced from inside the housing to outside the housing, and therefore when not in use, the puncture needle can be stored in the housing, and when in use, the puncture needle can be advanced to outside the housing. Therefore, when not in use, exposure of the puncture needle can be prevented, thereby achieving improvement in safety and prevention of damage to the puncture needle.
Furthermore, because the second substrate is fixed and the first substrate advances, a simple structure can be achieved.

### EFFECTS OF THE INVENTION

With a circuit board for body fluid collection of the present invention, assembling procedures of components can be reduced. Furthermore, the size of the device can be decreased. Furthermore, occurrence of wiring disconnection can be reduced, and an improvement in measurement reliability can be achieved.
Also, with a biosensor according to the present invention, improvement in safety, prevention of puncture needle damage, and structure simplification can be achieved.

### BRIEF DESCRIPTION OF THE DRAWINGS

[FIG. 1] FIG. 1 shows a circuit board for blood collection as a first embodiment of a circuit board for body fluid collection of the present invention: (a) is a plan view thereof; (b) is a bottom view thereof; and (c) is a cross-sectional view taken along line A-A in (a).
[FIG. 2] FIG. 2 is a process drawing for illustrating an example of a method for producing a wired circuit board for blood collection in the first embodiment: (a) illustrates a step of preparing a metal substrate; (b) illustrates a step of forming an insulating base layer; (c) illustrates a step of forming a conductive pattern; (d) illustrates a step of forming an insulating cover layer 5; (e) illustrates a step of trimming the metal substrate; and (f) illustrates a step of applying a chemical agent to an electrode.
[FIG. 3] FIG. 3 shows a glucose sensor as a first embodiment of a biosensor of the present invention, in which the circuit board for blood collection of FIG. 1 is mounted: (a) is a schematic perspective view thereof; and (b) to (c) are side sectional views for describing a method for its use.
[FIG. 4] FIG. 4 shows a circuit board for blood collection as a second embodiment of a circuit board for body fluid collection of the present invention: (a) is a plan view thereof; (b) is a bottom view thereof; and (c) is a cross-sectional view taken along line B-B in (a).
[FIG. 5] FIG. 5 shows a glucose sensor as a second embodiment of a biosensor of the present invention, in which the circuit board for blood collection of FIG. 4 is mounted: (a) is a schematic perspective view thereof; and (b) to (c) are side sectional views for describing a method for its use.
[FIG. 6] FIG. 6 shows a circuit board for blood collection as a third embodiment of a circuit board for body fluid collection of the present invention: (a) is a plan view thereof; (b) is a bottom view thereof; and (c) is a cross-sectional view taken along line C-C in (a).
[FIG. 7] FIG. 7 shows a glucose sensor as embodiment 3-1 of a biosensor of the present invention, in which the circuit board for blood collection of FIG. 6 is mounted: (a) is a schematic perspective view thereof; and (b) to (c) are side sectional views for describing a method for its use.
[FIG. 8] FIG. 8 shows a glucose sensor as embodiment 3-2 of a biosensor of the present invention, in which the circuit board for blood collection of FIG. 6 is mounted: (a) is a schematic perspective view thereof; and (b) to (c) are side sectional views for describing a method for its use.
[FIG. 9] FIG. 9 shows a circuit board for blood collection as a fourth embodiment of a circuit board for body fluid collection of the present invention: (a) is a plan view thereof; (b) is a bottom view thereof; and (c) is a cross-sectional view taken along line C-C in (a).
[FIG. 10] FIG. 10 shows a glucose sensor as a fourth embodiment of a biosensor of the present invention, in which the circuit board for blood collection of FIG. 9 is mounted: (a) is a schematic perspective view thereof; and (b) to (c) are side sectional views for describing a method for its use.

### EMBODIMENT OF THE INVENTION

### (First Embodiment)

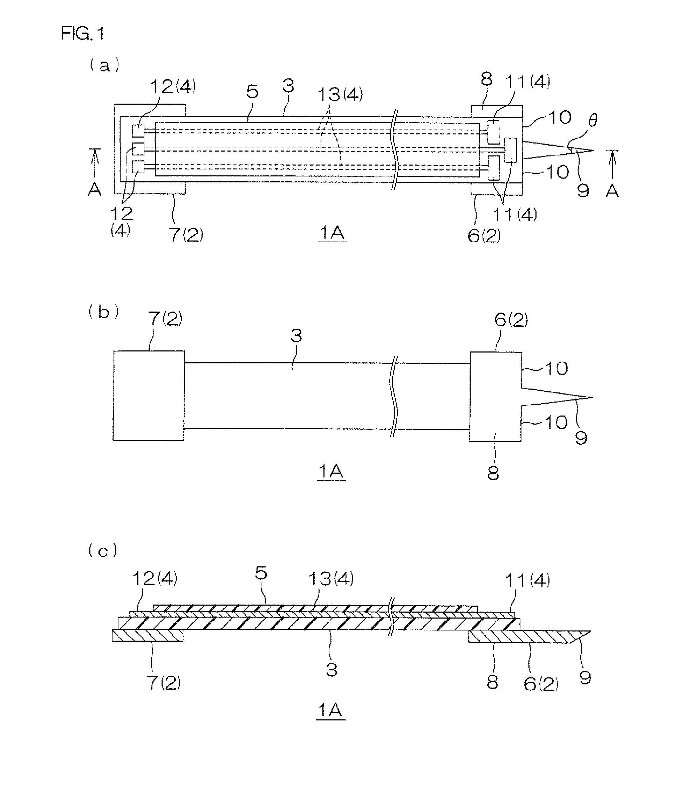

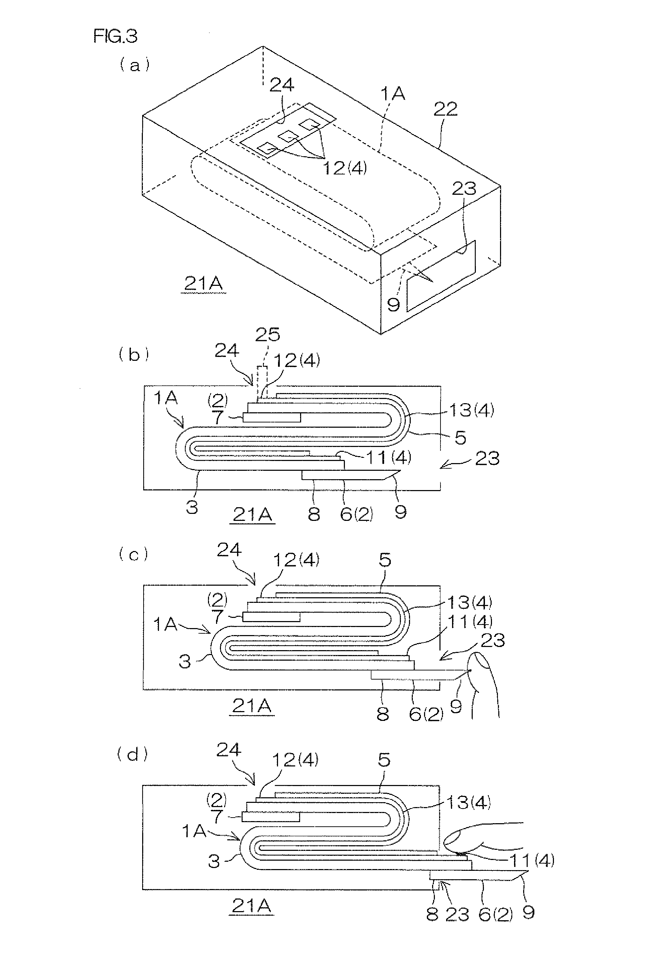

FIG. 1 shows a circuit board for blood collection as a first embodiment of a circuit board for body fluid collection of the present invention: (a) is a plan view thereof; (b) is a bottom view thereof; and (c) is a cross-sectional view taken along line A-A in (a).
A circuit board for body fluid collection 1A shown in FIG. 1 is mounted in a glucose sensor 21A (ref: FIG. 3) as a biosensor for a patient to puncture skin of, for example, a finger to collect blood, and measure a glucose level in the collected blood.

The circuit board for body fluid collection 1A includes, as shown in FIG. 1 (c), a metal substrate 2; an insulating base layer 3 as a flexible insulation layer formed on the metal substrate 2; a conductive pattern 4 formed on the insulating base layer 3; and an insulating cover layer 5 formed on the insulating base layer 3 so as to cover the conductive pattern 4.
The metal substrate 2 is formed of a metal foil or a metal thin plate. Examples of metal materials that may be used to form the metal substrate 2 include nickel, chromium, iron, and stainless steel (SUS304, SUS430, and SUS316L). Preferably, stainless steel is used.

The thickness of the metal substrate 2 is, for example, 10 to 300 µm, or preferably 20 to 100 µm. When the thickness is below 10 µm, the skin puncturing may not be performed because of insufficient strength. On the other hand, when the thickness exceeds 300 µm, the puncturing may cause pain and damage the skin excessively.
The metal substrate 2 includes a first substrate 6 and a second substrate 7. The first substrate 6 is provided integrally with, as shown in FIG. 1 (a), a needle support portion 8 that extends in width directions (in the following, referred to as width direction) perpendicular to the longitudinal direction (in the following, referred to as longitudinal direction) of the circuit board for body fluid collection 1A and has a generally rectangular flat plate shape when viewed from above; and a puncture needle 9 that protrudes from the needle support portion 8.

The puncture needle 9 is provided so as to protrude along the longitudinal direction continuously, from a widthwise center of a lengthwise one end face of the needle support portion 8. The puncture needle 9 is formed into a generally triangular shape (isosceles triangle) with its distal end tapered to form an acute angle when viewed from above. Angle θ of the distal end is, for example, 10 to 30°, or preferably 15 to 25°. When angle θ of the distal end is below 10°, the skin puncturing may not be performed because of insufficient strength. On the other hand, when angle θ exceeds 30°, the puncturing may become difficult. The length of the puncture needle 6 in the longitudinal direction is, for example, 0.5 to 5 mm.

The lengthwise one end face (that is, an end face of the needle support portion 8 at a downstream side in the puncturing direction) of the needle support portion 8 extends from the proximal end of the puncture needle 9 in both width directions, and serves as a stopper 10 for restricting further puncturing with the puncture needle 9. That is, the stopper 10 is disposed at an end portion of the puncture needle 9 at a downstream side in the puncturing direction, and the protruding length of the stopper 10 from the puncture needle 9 in both width directions is, for example, 0.1 to 2 mm.

The second substrate 7 is disposed at an upstream side in the puncturing direction (left side in FIG. 1) relative to the first substrate 6 with a space provided therebetween. The second substrate 7 is formed into a generally rectangular flat plate shape extending in width directions when viewed from above. The length of the second substrate 7 in the longitudinal direction is longer than that of the first substrate 6, and the length of the second substrate 7 in the width direction is the same as that of the first substrate 6. The space between the first substrate 6 and the second substrate 7 is appropriately selected according to the size, use, and purpose of the blood-sugar-level measuring device 31A.

The insulating base layer 3 is provided so as to extend between the first substrate 6 and the second substrate 7.
The insulating base layer 3 is formed of an insulative flexible film. Examples of insulation materials that form the insulating base layer 3 include synthetic resins such as polyimide resin, polycarbonate resin, polyethylene resin, polyethyleneterephthalate resin, epoxy resin, and fluorocarbon resin. In view of mechanical durability, and chemical resistance, polyimide resin is preferably used.

The thickness of the insulating base layer 3 is, for example, 3 to 50 µm, or preferably 5 to 25 µm. When the thickness is below 3 µm, there may be a case where an insulation defect such as pinholes is caused. On the other hand, when the thickness exceeds 50 µm, cutting and trimming may become difficult due to poor flexibility.
The insulating base layer 3 is formed into a rectangular shape extending in the longitudinal direction when viewed from above. The insulating base layer 3 is formed so as to have a slightly narrower length in the width direction than that of the metal substrate 2. An end portion at an upstream side in the puncturing direction of the insulating base layer 3 is laminated onto the second substrate 6 so as to cover an upper face of the second substrate 6. An end portion at a downstream side in the puncturing direction of the insulating base layer 3 is laminated onto the first substrate 7 so as to cover an upper face of the first substrate 7. Although an end edge of the insulating base layer 3 at a downstream side in the puncturing direction is disposed so as to coincide with that of the first substrate 6 in FIG. 1 when viewed from above, the end edge may also be formed so as to cover that of the first substrate 6. By covering the end edge of the first substrate 6 at a downstream side in the puncturing direction with the end edge of the insulating base layer 3 at a downstream side in the puncturing direction, that is, by covering the stopper 10, the contact of the skin can be cushioned against the stopper 10 at the time of puncturing.

The conductive pattern 4 integrally includes three electrodes 11, three terminals 12, and three wirings 13.
The conductive pattern 4 is formed of a conductive foil such as metal foil. Examples of conductive materials that form the conductive pattern 4 include metal materials such as iron, nickel, chromium, copper, gold, silver, platinum, and alloys thereof. The conductive material is selected appropriately in view of adhesiveness to the insulating base layer 3 and the insulating cover layer 5, and easy workability. Two or more conductive materials may be laminated as well.

The thickness of the conductive pattern 4 is, for example, 5 to 50 µm, or preferably 10 to 20 µm.
The three electrodes 11 are disposed so as to overlap with the first substrate 6 when projected in the thickness direction of the conductive pattern 4. To be specific, three electrodes 11 are disposed on the insulating base layer 3 corresponding to the needle support portion 8. These electrodes 11 are formed into a generally rectangular shape extending in the width direction when viewed from above, and two of them are disposed in parallel with a space provided therebetween in the width direction, and the other one is disposed between the two of them at a downstream side in the puncturing direction relative to the two of them.

The three electrodes 11 correspond to a working electrode, a counter electrode, and a reference electrode, respectively. The length of a side of the electrode 11 is, for example, 100 µm to 2.5 mm.
The three electrodes 11 are disposed within the range of, for example, 0.2 to 5 mm, or preferably 0.5 to 3 mm from the distal end of the puncture needle 6 in the puncturing direction. When the distance between the distal end of the puncture needle 6 and the electrodes 11 is too short, the electrodes 11 sting the skin along with the puncture needle 6, and a chemical agent 16 (described later) applied to the surface of the electrodes 11 may be dispersed into the body, which may hinder accurate measurements. On the other hand, when the space between the distal end of the puncture needle 6 and the electrodes 11 is too long, a structure for utilizing aspiration or capillarity to introduce blood from the puncture needle 6 to the electrodes 11 becomes necessary.

The three terminals 12 are provided so as to correspond to the three electrodes 11, and are disposed so as to overlap with the second substrate 7 when projected in the thickness direction of the conductive pattern 4. To be specific, the three terminals 12 are disposed on the insulating base layer 3 corresponding the second substrate 7. These terminals 12 are formed into a generally rectangular shape (generally square) that is slightly smaller than the electrodes 11 when viewed from above. The three terminals 12 are disposed in parallel in the width direction with a space provided therebetween.

The three wirings 13 are disposed on the first substrate 6, the second substrate 7, and the insulating base layer 3 that is between these substrates. The three wirings 13 are disposed in parallel in the width direction with a space provided therebetween, and are provided along the longitudinal direction so as to electrically connect the electrodes 11 and the corresponding terminals 12.
The electrodes 11, corresponding terminals 12, and wirings 13 connected thereto are provided continuously and integrally. The length of the wirings 13 in the width direction is, for example, 0.01 to 2 mm, and the length of the wirings 13 in the longitudinal direction is appropriately selected according to the length of the insulating base layer 3 in the longitudinal direction.

The insulating cover layer 5 is provided on the insulating base layer 3 so as to cover the wirings 13. To be specific, the end edge of the insulating cover layer 5 at a downstream side in the puncturing direction are formed straight along the width direction at an upstream side of the three electrodes 11 in the puncturing direction so as to expose the three electrodes 11. The end edge of the insulating cover layer 5 at an upstream side in the puncturing direction is formed straight along the width direction at a downstream side of the three terminals 12 in the puncturing direction so as to expose the three terminals 12.

As the insulation materials that form the insulating cover layer 5, the above-described insulation materials for the insulating base layer 3 are used. The thickness of the insulating cover layer 5 is, for example, 2 to 50 µm.

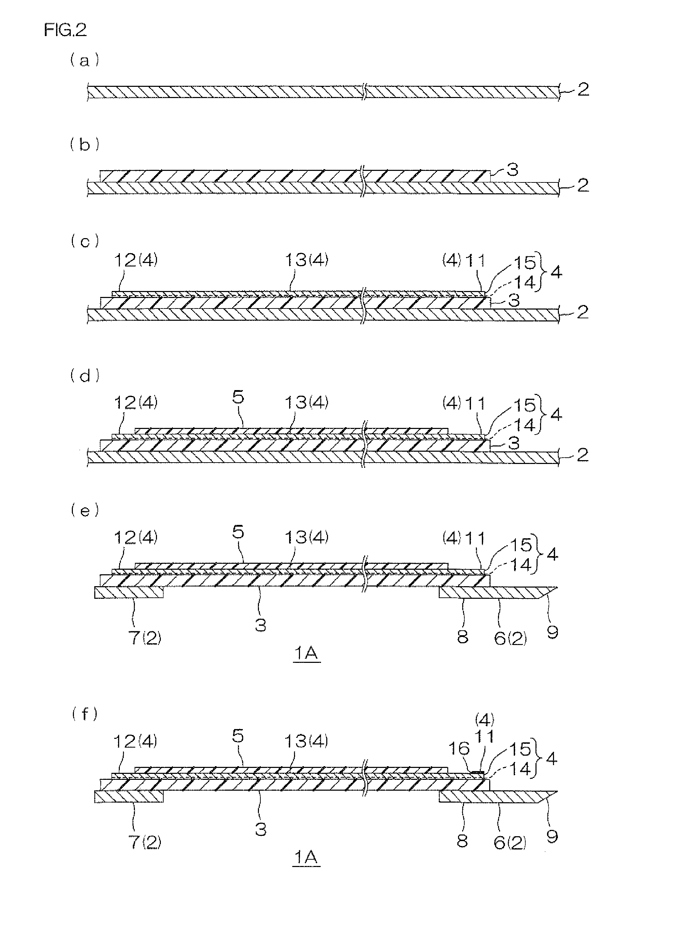

FIG. 2 is a process drawing for illustrating an example of a method for producing a wired circuit board for blood collection 1A. Next, with reference to FIG. 2, a method for producing the wired circuit board for blood collection 1A is described.
The wired circuit board for blood collection 1A can be produced in the same manner as the known method for producing a flexible printed wired circuit board with a reinforcing plate provided therewith.

That is, in this method, first, as shown in FIG. 2 (a), a metal substrate 2 is prepared. The metal substrate 2 is prepared, for example, as an elongated metal foil that ensures a large number of the metal substrate 2. From such an elongated metal foil, a plurality of wired circuit boards for blood collection 1A are produced by trimming the metal substrates 2 (described later).
Next, in this method, as shown in FIG. 2 (b), an insulating base layer 3 is formed on the surface of the metal substrate 2. For the formation of the insulating base layer 3, for example, the following methods are used: a method in which a varnish of a photosensitive synthetic resin is applied on the surface of the metal substrate 2, photoprocessed, and then cured; a method in which a synthetic resin film is laminated onto the surface of the metal substrate 2, an etching resist having the same pattern as that of the insulating base layer 3 is laminated onto the surface of the film, and afterwards, the film exposed from the etching resist is wet-etched; a method in which a synthetic resin film that is punched by a machine in advance is laminated onto the surface of the metal substrate 2; and a method in which a synthetic resin film is laminated onto the surface of the metal substrate 2 first, and then subjected to discharge processing or laser processing. In view of processing accuracy, the method in which a varnish of a photosensitive synthetic resin is applied on the surface of the metal substrate 2, photoprocessed, and then cured is preferably used.

Afterwards, in this method, as shown in FIG. 2 (c), a conductive pattern 4 is formed. For the formation of the conductive pattern 4, a known patterning method for forming printed wirings is used, such as an additive method and a subtractive method. In view of achieving formation of a minute pattern, the additive method is preferably used. In the additive method, for example, a metal thin film 14 is formed on the surface of the insulating base layer 3 by chemical vapor deposition or sputtering, and after a plating resist is formed on the surface of the metal thin film 14, a plating layer 15 is formed on the surface of the metal thin film 14 exposing from the plating resist by electrolytic plating using the metal thin film 14 as a seed film. The conductive pattern 4 is formed from the metal thin film 14 and the plating layer 15 in this manner.

The conductive pattern 4 can also be formed only of the metal thin film 14 by chemical vapor deposition or sputtering.
Upon formation of the conductive pattern 4, a different type of metal plating layer may also be formed on the surface of the electrodes 11 and the surface of the terminals 12 by further electrolytic plating or electroless plating.
Then, in this method, as shown in FIG. 2 (d), an insulating cover layer 5 is formed.
For the formation of the insulating cover layer 5, the same method as the method for forming the insulating base layer 3 is used. A preferable method that may be used is the method in which a varnish of a photosensitive synthetic resin is applied on the surface of the insulating base layer 3 so as to expose the electrodes 11 and the terminals 12, and to cover the wirings 13, photoprocessed, and then cured.

Afterwards, as shown in FIG. 2 (e), the metal substrate 2 is trimmed. For the trimming of the metal substrate 2, for example, discharge processing, laser processing, mechanical punching processing, or etching processing is used. In view of easy cleaning after processing, etching processing (wet etching) is preferably used.
In this manner, the wired circuit board for blood collection 1A can be obtained. In the obtained wired circuit board for blood collection 1A, as shown in FIG. 2 (f), a chemical agent 16 is applied on the electrodes 11: that is, for example, glucose oxidase, or glucose dehydrogenase as an enzyme, and for example, potassium ferricyanide, ferrocene, or benzoquinone as a mediator, alone or in combination is applied. For the coating of the chemical agent 16, there is used an appropriate method such as, for example, a dipping method, a spray method, or an inkjet method.

Depending on the type of the chemical agent 16, after the plating layer of a different metal is formed on the surface of the electrodes 11 as described above, it is also possible to further form a coating of a different metal in advance, and provide a predetermined potential difference therebetween. To be specific, for example, after a gold plating layer is formed, silver or silver chloride is applied on the surface of the gold plating layer.
FIG. 3 shows a glucose sensor as a first embodiment of a biosensor of the present invention, in which the circuit board for blood collection of FIG. 1 is mounted: (a) is a schematic perspective view thereof; and (b) to (c) are side sectional views for describing a method for its use.

As shown in FIG. 3 (a), a glucose sensor 21A is prepared as a disposable type that is discarded once the measurement is performed. The glucose sensor 21A is provided with a housing 22, and the wired circuit board for blood collection 1A accommodated in the housing 22.
The housing 22 is formed into a box shape extending in the longitudinal direction. In a side wall (front wall) of the housing 22 at a downstream side in the puncturing direction, a front-side opening 23 having a rectangular shape elongated in the width direction is formed. In an upper wall of the housing 22, an upper-side opening 24 having a rectangular shape elongated in the width direction is formed at an upstream side in the puncturing direction.

In the housing 22, the wired circuit board for blood collection 1A is disposed so as to be generally S-shaped when viewed from a side, the second substrate 7 at the top and the first substrate 6 at the bottom.
The second substrate 7 is disposed in the housing 22 at an upstream side in the puncturing direction so as to vertically face the upper-side opening 24, and the terminals 12 are exposed from the upper-side opening 24. The second substrate 7 is fixed at such a position to a frame member (not shown) provided in the housing 22.

The first substrate 6 is disposed in the housing 22 at a downstream side in the puncturing direction so as to face the front-side opening 23 in the longitudinal direction, and the puncture needle 9 is exposed from the front-side opening 23. The first substrate 6 is supported by a sliding member (not shown) provided in the housing 22. The sliding member slides along the longitudinal direction by a known mechanism.
By such sliding, the first substrate 6 slides along the longitudinal direction, through an accommodated position (ref: FIG. 3(b)) at which the puncture needle 9 and the electrodes 11 are disposed in the housing 22; a puncturing position (ref: FIG. 3(c)) at which the puncture needle 9 is disposed outside the housing 22 and the electrodes 11 are disposed inside the housing 22; and a blood collection position (ref: FIG. 3(d)) at which the puncture needle 9 and the electrodes 11 are disposed outside the housing 22.

When the glucose sensor 21A is not in use, the first substrate 6 is disposed at the accommodated position, as shown in FIG. 3 (b).
When in use, the first substrate 6 slides toward a downstream side in the puncturing direction, as shown in FIG. 3 (c), to be disposed at the puncturing position, and allows a patient to puncture, for example, his/her finger with the puncture needle 6 to cause a trace amount of bleeding. At this time, when a stopper 10 is brought in contact with the skin, further puncturing with the puncture needle 9 is restricted.

Afterwards, the first substrate 6 further slides toward a downstream side in the puncturing direction, as shown in FIG. 3 (d) to be disposed at the blood collection position, and the patient himself/herself brings the punctured portion into contact with the electrodes 11. Then, a chemical agent 16 at the surface of the electrodes 11 is allowed to react with the blood, and the resistance value when a voltage is applied to the electrodes 11 changes according to the blood-sugar level in the blood.
Next, the first substrate 6 slides toward an upstream side in the puncturing direction, as shown in FIG. 3 (b) to be disposed at the accommodated position again, and accommodated in the housing 22.

Then, external terminals 25 of a blood-sugar-level measuring device (not shown) as an external measurement device including a CPU and a display are connected to the terminals 12. The blood-sugar-level measuring device is a device for easily measuring a blood-sugar level, and has the same structure as that of various commercially available known devices.
Then, in the blood-sugar-level measuring device, the CPU applies a voltage to the terminals 12, and measures the glucose level based on the changes in the resistance value. The measured glucose level is displayed at the display as the blood-sugar level.

In this circuit board for body fluid collection 1A, a conductive pattern 4 that is integrally provided with the electrodes 11, the terminals 12, and the wirings 13 is provided on the insulating base layer 3 that extends between the first substrate 6 having the puncture needle 9 and the second substrate 7. Therefore, assembling procedures of components can be reduced. Also, the size of the device can be reduced. Furthermore, the wirings 12 are provided on the insulating base layer 3 as the conductive pattern 4. Therefore, occurrence of disconnection can be reduced, and improvement in measurement reliability can be achieved.

Terminals 12 are disposed on the insulating base layer 3 corresponding to the second substrate 7. Therefore, the terminals 12 can be reliably supported. As a result, connection reliability between the terminal 12 and the blood-sugar-level measuring device electrically connected thereto can be improved, and an improvement in measurement reliability can be achieved.
The electrodes 11 are disposed on the insulating base layer 3 corresponding to the needle support portion 8. Therefore, the electrodes 11 can be disposed in the proximity of the puncture needle 9. As a result, the blood that was caused to be bled by the puncturing with the puncture needle 9 can be bought into contact with the electrodes 11 easily. As a result, a glucose level in blood can be measured easily.

In this glucose sensor 21A, while the second substrate 7 is fixed, the first substrate 6 can be slid along the longitudinal direction, through the accommodated position, the puncturing position, and the blood collection position. Therefore, when not in use, exposure of the puncture needle 9 is prevented, which allows an improvement in safety and prevention of damage to the puncture needle 9. Furthermore, because the second substrate 7 is fixed and the first substrate 6 slides, a simple structure can be achieved.

### (Second Embodiment)

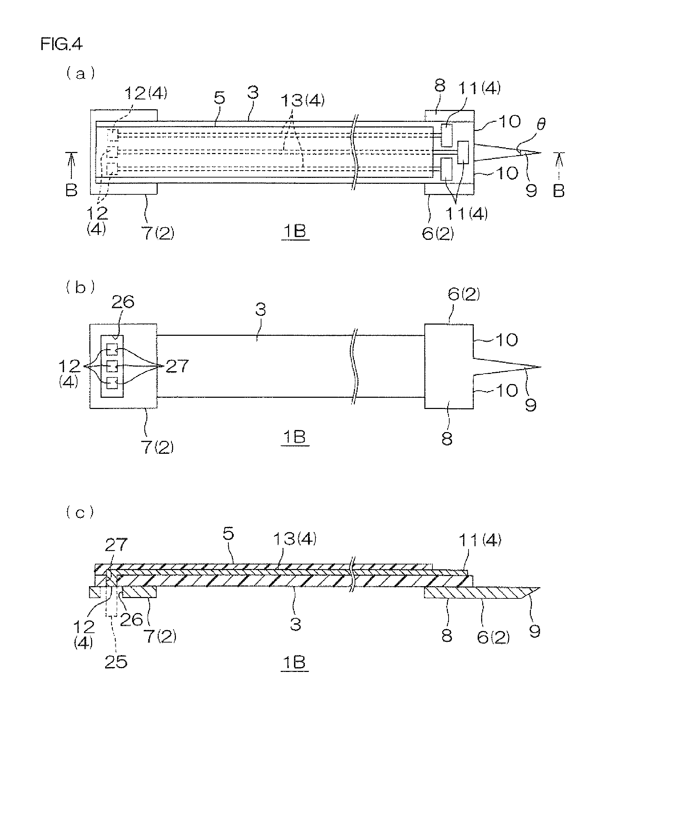

FIG. 4 shows a circuit board for blood collection as a second embodiment of a circuit board for body fluid collection of the present invention: (a) is a plan view thereof; (b) is a bottom view thereof; and (c) is a cross-sectional view taken along line B-B in (a).

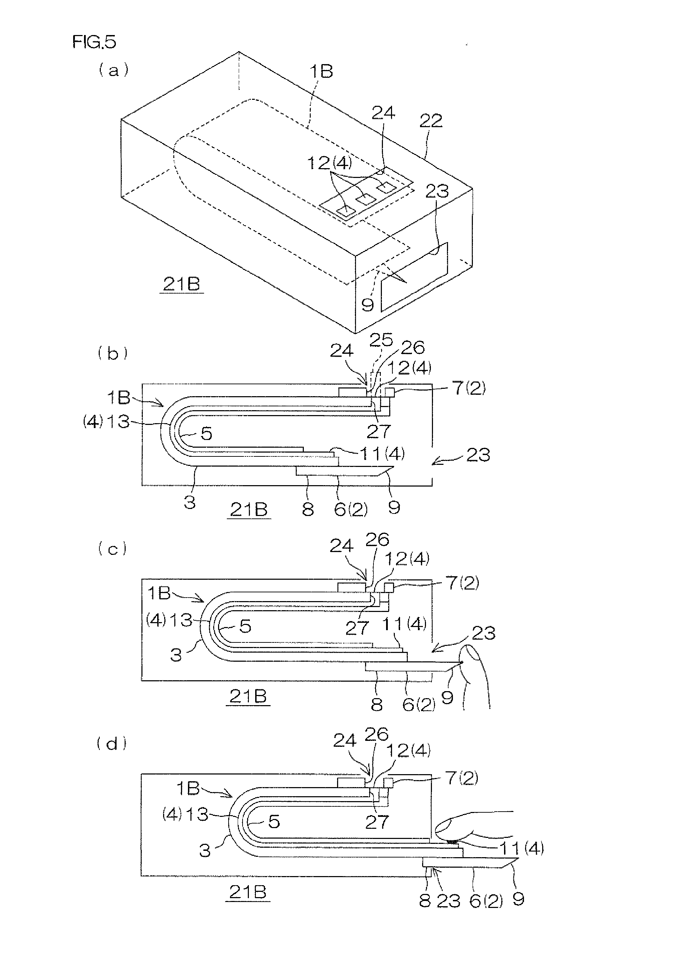

FIG. 5 shows a glucose sensor as a second embodiment of a biosensor of the present invention, in which the circuit board for blood collection of FIG. 4 is mounted: (a) is a schematic perspective view thereof; and (b) to (c) are side sectional views for describing a method for its use.
In FIGs. 4 and 5, the same members as shown in FIGs. 1 and 3 are given the same reference numerals, and descriptions thereof are omitted.

In FIG. 4, in this circuit board for blood collection 1B, the three terminals 13 are exposed from the lower face of the second substrate 7.
That is, in the second substrate 7, a substrate-side opening 26 having a generally rectangular shape and extending in the width direction is formed at an upstream side in the puncturing direction, so as to pierce through in the thickness direction. Also, in the insulating base layer 3, base-side openings 27 corresponding to the terminal portions 13 are formed so as to face within the substrate-side opening 26, and to pierce through in the thickness direction.

The terminal portions 13 are formed so as to fill in the base-side openings 27. In this fashion, the terminal portions 13 face within the substrate-side openings 26, and exposed from the lower face of the second substrate 7.
The insulating cover layer 5 is positioned so as to cover the terminal portions 13, and such that the end edge thereof at an upstream side in the puncturing direction coincides with that of the insulating base layer 3 when viewed from above.

The circuit board for blood collection 1B in the second embodiment can be produced in the same manner as the circuit board for blood collection 1A in the first embodiment. The base-side openings 27 are formed when, for example, the insulating base layer 3 is photoprocessed, and the substrate-side opening 26 is formed, for example, by etching simultaneously with trimming of the metal substrate 2.
In this circuit board for blood collection 1B, the three terminals 13 are exposed from the lower face of the second substrate 7, and therefore external terminals 25 of a blood-sugar-level measuring device (not shown) can be connected from below. Therefore, it can be used effectively when the external terminals 25 cannot be connected from above.

In this glucose sensor 21B including this circuit board for blood collection 1B, the wired circuit board for blood collection 1B is disposed in the housing 22, as shown in FIG. 5 (a), so as to be generally U-shaped when viewed from a side, the second substrate 7 at the top and the first substrate 6 at the bottom.
In this glucose sensor 21B, the upper-side opening 24 is formed in the upper wall of the housing 22 at a downstream side in the puncturing direction. The second substrate 7 is fixed to a frame member (not shown) so that the terminals 12 are exposed from the upper-side opening 24.

The first substrate 6 is disposed in the housing 22 at a downstream side in the puncturing direction so as to face the front-side opening 23 in the longitudinal direction, and the puncture needle 9 is exposed from the front-side opening 23. The first substrate 6 is supported by a sliding member (not shown) provided in the housing 22. The sliding member slides along the longitudinal direction by a known mechanism.
By such sliding, the first substrate 6 slides along the longitudinal direction, through the accommodated position (ref: FIG. 5(b)) at which the puncture needle 9 and the electrodes 11 are disposed in the housing 22; the puncturing position (ref: FIG. 5(c)) at which the puncture needle 9 is disposed outside the housing 22 and the electrodes 11 are disposed inside the housing 22; and the blood collection position (ref: FIG. 5(d)) at which the puncture needle 9 and the electrodes 11 are disposed outside the housing 22.

When the glucose sensor 21B is not in use, the first substrate 6 is disposed at the accommodated position, as shown in FIG. 5 (b).
When in use, the first substrate 6 slides toward a downstream side in the puncturing direction, as shown in FIG. 5 (c), to be disposed at the puncturing position, and allows a patient to puncture, for example, his/her finger with the puncture needle 6 to cause a trace amount of bleeding.

Afterwards, the first substrate 6 further slides toward a downstream side in the puncturing direction, as shown in FIG. 5 (d) to be disposed at the blood collection position, and the patient himself/herself brings the punctured portion into contact with the electrodes 11. Then, the chemical agent 16 at the surface of the electrodes 11 is allowed to react with the blood, and the resistance value when a voltage is applied to the electrodes 11 changes according to the blood-sugar level in the blood.
Next, the first substrate 6 slides toward an upstream side in the puncturing direction, as shown in FIG. 5 (b) to be disposed at the accommodated position again, and accommodated in the housing 22. Thereafter, external terminals 25 of a blood-sugar-level measuring device (not shown) are connected, and a glucose level is measured based on the changes in the resistance value. The measured glucose level is displayed at the display as the blood-sugar level.

In this glucose sensor 21B, the wired circuit board for blood collection 1B is disposed in the housing 22, so as to be generally U-shaped when viewed from a side. That is, the wired circuit board for blood collection 1B can be accommodated in the housing 22 by bending the insulating base layer 3 at one portion, and therefore a size reduction of the glucose sensor 21B can be achieved.

### (Third Embodiment)

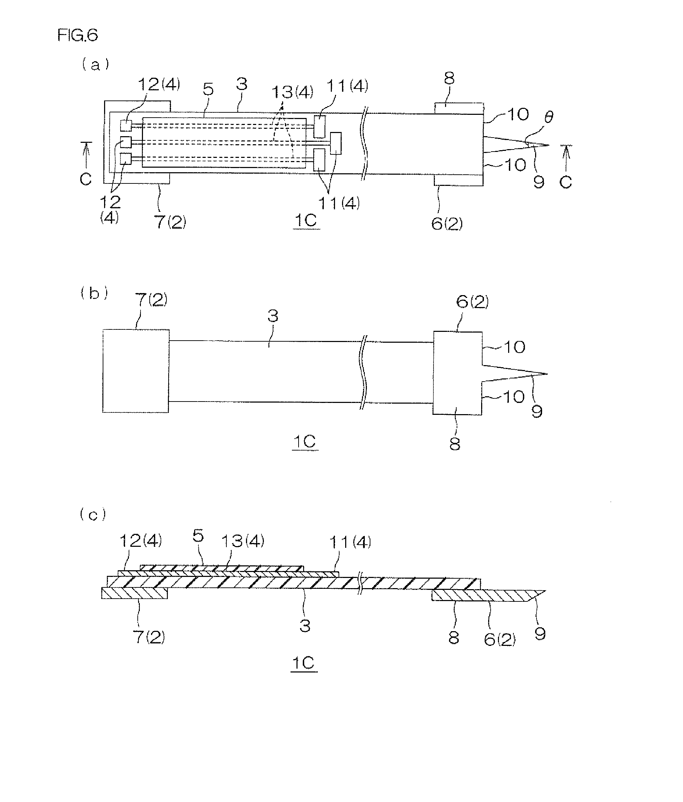

FIG. 6 shows a circuit board for blood collection as a third embodiment of a circuit board for body fluid collection of the present invention: (a) is a plan view thereof; (b) is a bottom view thereof; and (c) is a cross-sectional view taken along line C-C in (a).

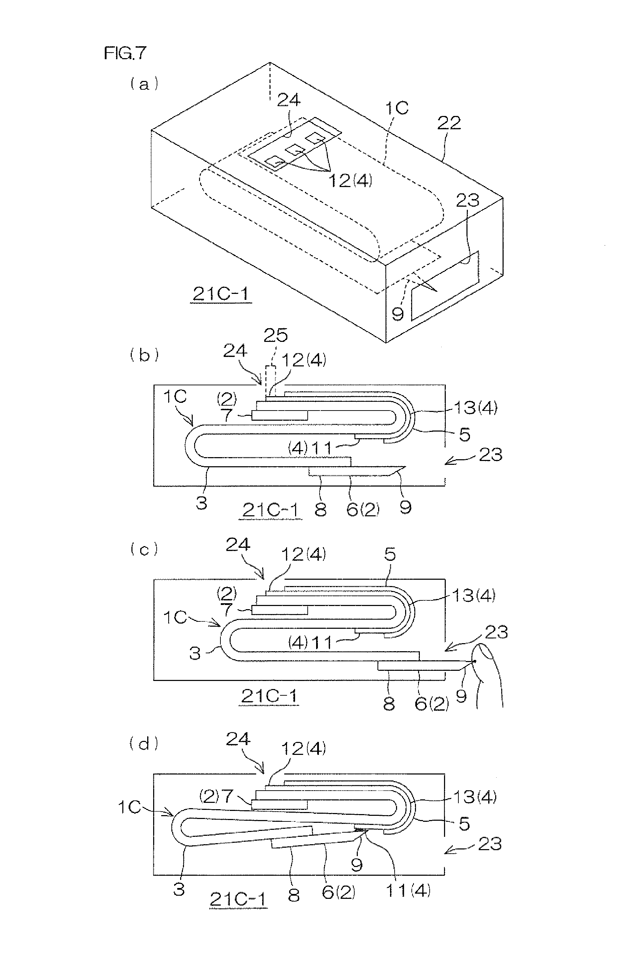

FIG. 7 shows a glucose sensor as embodiment 3-1 of a biosensor of the present invention, in which the circuit board for blood collection of FIG. 6 is mounted: (a) is a schematic perspective view thereof; and (b) to (c) are side sectional views for describing a method for its use.
In FIGs. 6 and 7, the same members as shown in FIGs. 1 and 3 are given the same reference numerals, and descriptions thereof are omitted.

In FIG. 6, in this circuit board for blood collection 1C, the three electrodes 11 are disposed, not on the insulating base layer 3 corresponding to the needle support portion 8, but on the insulating base layer 3 that is disposed between the first substrate 6 and the second substrate 7.
That is, only the insulating base layer 3 is formed on the first substrate 6, and the three electrodes 11 are provided on the insulating base layer 3 that is at an upstream side of the first substrate 6 in the puncturing direction and at a downstream side of the second substrate 7 in the puncturing direction. The positions of the three electrodes 11 along the longitudinal direction are appropriately selected.

In corresponding with the three electrodes 11, the end edge of the insulating cover layer 5 at a downstream side in the puncturing direction is disposed between the first substrate 6 and the second substrate 7, so as to expose the three electrodes 11.
The circuit board for blood collection 1C of the third embodiment can be produced in the same manner as the circuit board for blood collection 1A of the first embodiment.
In this glucose sensor 21C-1 including this circuit board for blood collection 1C, the wired circuit board for blood collection 1C is disposed in the housing 22, as is the case with the wired circuit board for blood collection 1A, as shown in FIG. 7 (a), so as to be generally S-shaped when viewed from a side, the second substrate 7 at the top and the first substrate 6 at the bottom.

The second substrate 7 is fixed, as is the case with the wired circuit board for blood collection 1A, to a frame member (not shown) so that the terminals 12 are exposed from the upper-side opening 24.
The first substrate 6 is disposed in the housing 22 at a downstream side in the puncturing direction so as to face the front-side opening 23 in the longitudinal direction, and the puncture needle 9 is exposed from the front-side opening 23. The first substrate 6 is supported by a sliding member (not shown) provided in the housing 22. The sliding member slides along the longitudinal direction by a known mechanism.

By such sliding, the first substrate 6 slides along the longitudinal direction, through the accommodated position (ref: FIG. 7(b)) at which the puncture needle 9 and the electrodes 11 are disposed in the housing 22, and the puncturing position (ref: FIG. 7(c)) at which the puncture needle 9 is disposed outside the housing 22 and the electrodes 11 are disposed inside the housing 22.
In the glucose sensor 21C-1, a pressing member (not shown) that can press the first substrate 6 upward in the accommodated position is provided. The pressing member includes, for example, a spring member that presses the first substrate 6 at appropriate timing.

When the glucose sensor 21C-1 is not in use, the first substrate 6 is disposed at the accommodated position, as shown in FIG. 7 (b).
When in use, the first substrate 6 slides toward a downstream side in the puncturing direction, as shown in FIG. 7 (c), to be disposed at the puncturing position, and allows a patient to puncture, for example, his/her finger with the puncture needle 6 to cause a trace amount of bleeding.

Afterwards, the first substrate 6 is disposed again at the accommodated position, as shown in FIG. 7 (d), to be disposed in the housing 22. At this time, the first substrate 6 is urged upward by pressing of the pressing member, so as to bring the puncture needle 9 into contact with the electrodes 11. Then, the chemical agent 16 at the surface of the electrodes 11 is allowed to react with the blood, and the resistance value when a voltage is applied to the electrodes 11 changes according to the blood-sugar level in the blood.

Thereafter, as shown in FIG. 7 (b), external terminals 25 of a blood-sugar-level measuring device (not shown) are connected, and a glucose level is measured based on the changes in the resistance value. The measured glucose level is displayed at the display as the blood-sugar level.
In the circuit board for body fluid collection 1C, by bending the insulating base layer 3, the puncture needle 9 can be brought into contact with the electrodes 11. Therefore, the blood adhered to the puncture needle 9 after the puncturing can be brought into contact with the electrodes 11. As a result, a glucose level in blood can be measured easily.

Furthermore, in the glucose sensor 21C-1, the puncture needle 9 is brought into contact with the electrodes 11 at the accommodated position. Therefore, the electrodes 11 are always disposed inside the housing 22, allowing prevention of contamination of the electrodes 11.

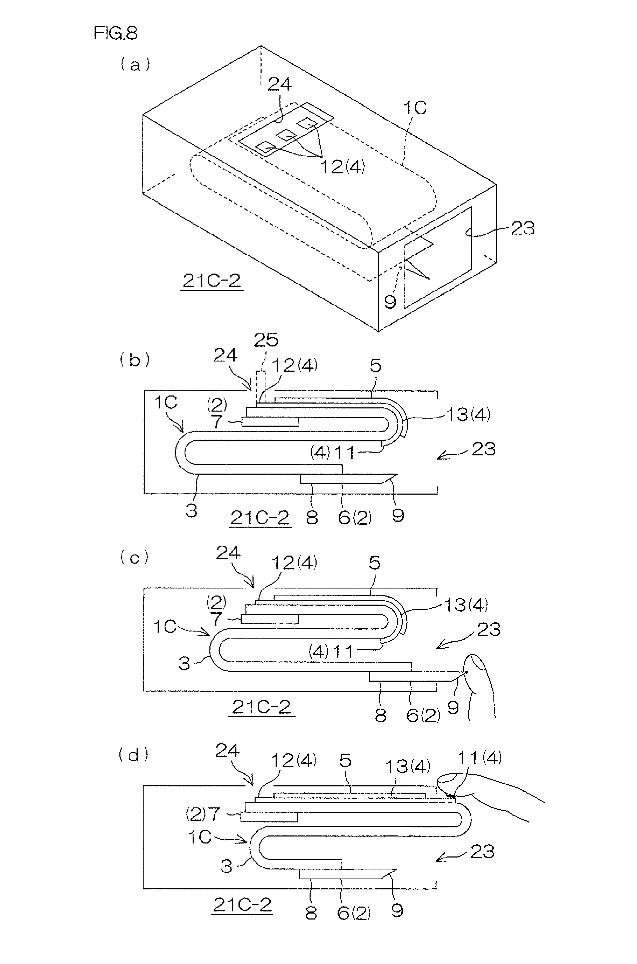

FIG. 8 shows a glucose sensor as embodiment 3-2 of a biosensor of the present invention, in which the circuit board for blood collection of FIG. 6 is mounted: (a) is a schematic perspective view thereof; and (b) to (c) are side sectional views for describing a method for its use.

In FIG. 8, the same members as shown in FIG. 3 are given the same reference numerals, and descriptions thereof are omitted.
As shown in FIG. 8, the circuit board for blood collection 1 of the third embodiment can be mounted in the glucose sensor 21C-2 of embodiment 3-2.
That is, in the glucose sensor 21C-2, the front-side opening 23 is formed to be larger in up and down directions than the above-described front-side opening 23.

The wired circuit board for blood collection 1C is disposed in the housing 22, as is the case with the wired circuit board for blood collection 1A, so as to be generally S-shaped when viewed from a side, the second substrate 7 at the top and the first substrate 6 at the bottom.
The second substrate 7 is fixed, as is the case with the wired circuit board for blood collection 1A, to a frame member (not shown) so that the terminals 12 are exposed from the upper-side opening 24.

The first substrate 6 is disposed in the housing 22 at a downstream side in the puncturing direction so as to face the front-side opening 23 in the longitudinal direction, and the puncture needle 9 is exposed from the front-side opening 23. The first substrate 6 is supported by a first sliding member (not shown) provided in the housing 22. The portion of the insulating base layer 3 where the electrodes 11 are disposed also is supported by a second sliding member (not shown) provided in the housing 22. The first sliding member and the second sliding member slide along the longitudinal direction by a known mechanism.

By such sliding, the first substrate 6 and the portion of the insulating base layer 3 where the electrodes 11 are disposed slide along the longitudinal direction, through the accommodated position (ref: FIG. 8(b)) at which the puncture needle 9 and the electrodes 11 are disposed in the housing 22, the puncturing position (ref: FIG. 8(c)) at which the puncture needle 9 is disposed outside the housing 22 and the electrodes 11 are disposed inside the housing 22, and the blood collection position (ref: FIG. 8(d)) at which the puncture needle 9 is disposed inside the housing 22 and the electrodes 11 are disposed outside the housing 22.

When the glucose sensor 21C-2 is not in use, the first substrate 6 is disposed at the accommodated position, as shown in FIG. 8 (b).
When in use, the first sliding member slides toward a downstream side in the puncturing direction, as shown in FIG. 8 (c) so that the first substrate 6 is disposed at the puncturing position, and allows a patient to puncture, for example, his/her finger with the puncture needle 6 to cause a trace amount of bleeding.

Afterwards, as shown in FIG. 8 (d), the first sliding member slides toward an upstream side in the puncturing direction, while the second sliding member slides toward a downstream side in the puncturing direction so that the first substrate 6 is disposed in the housing 22, and the electrodes 11 are disposed at the blood collection position, i.e., outside the housing 22. At the blood collection position, the electrodes 11 are exposed from the front-side opening 23, and therefore a patient himself/herself brings the punctured portion into contact with the electrodes 11. Then, the chemical agent 16 at the surface of the electrodes 11 is allowed to react with the blood, and the resistance value when a voltage is applied to the electrodes 11 changes according to the blood-sugar level in the blood.

Then, both of the first sliding member and the second sliding member slide toward an upstream side in the puncturing direction so that the first substrate 6 and the electrodes 11 are disposed again at the accommodated position, as shown in FIG. 8 (b), and accommodated in the housing 22.
Thereafter, in the same manner as in the above, external terminals 25 of a blood-sugar-level measuring device (not shown) are connected, and a glucose level is measured based on the changes in the resistance value. The measured glucose level is displayed at the display as the blood-sugar level.

Furthermore, in the glucose sensor 21C-2, the puncture needle 9 is accommodated in the housing 22 at the blood collection position, and therefore when a patient brings the punctured portion into contact with the electrodes 11, contact with the puncture needle 9 can be prevented, achieving an improvement in safety.

### (Fourth Embodiment)

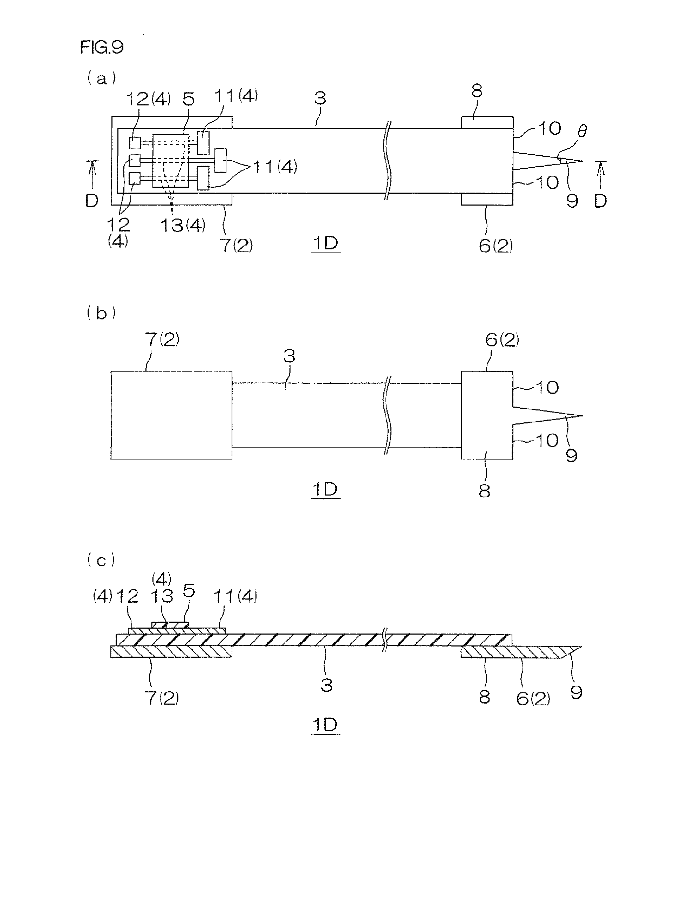

FIG. 9 shows a circuit board for blood collection as a fourth embodiment of a circuit board for body fluid collection of the present invention: (a) is a plan view thereof; (b) is a bottom view thereof; and (c) is a cross-sectional view taken along line C-C in (a).

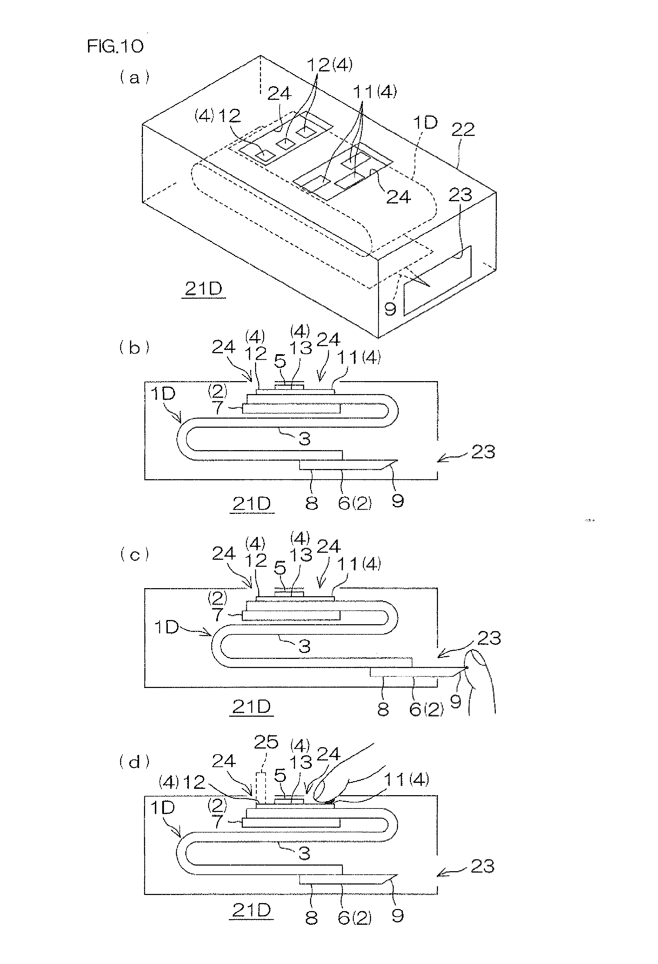

FIG. 10 shows a glucose sensor as a fourth embodiment of a biosensor of the present invention, in which the circuit board for blood collection of FIG. 9 is mounted: (a) is a schematic perspective view thereof; and (b) to (c) are side sectional views for describing a method for its use.
In FIGs. 9 and 10, the same members as shown in FIGs. 1 and 3 are given the same reference numerals, and descriptions thereof are omitted.

In this circuit board for blood collection 1D shown in FIG. 9, the three electrodes 11 are not disposed on the insulating base layer 3 corresponding to the needle support portion 8, but disposed on the insulating base layer 3 corresponding to the second substrate 7.
That is, only the insulating base layer 3 is formed on the first substrate 6, and the three electrodes 11 are provided on the insulating base layer 3 that is formed on the second substrate 7 that is at an upstream side of the first substrate 6 in the puncturing direction.

In correspondence with the three electrodes 11, the end edge of the insulating cover layer 5 at a downstream side in the puncturing direction is disposed only on the second substrate 7 so as to expose the three electrodes 11.
The circuit board for blood collection 1D of the fourth embodiment can be produced in the same manner as the circuit board for blood collection 1A of the first embodiment.
In the glucose sensor 21D of the fourth embodiment, two upper-side openings 24 are formed in the upper wall. One of the upper-side opening 24 is disposed at an upstream side in the puncturing direction, and the other of the upper-side opening 24 is disposed at a downstream side in the puncturing direction.

As shown in FIG. 10 (a), in the glucose sensor 21D including the circuit board for blood collection 1D, the wired circuit board for blood collection 1D is disposed in the housing 22, as is the case with the wired circuit board for blood collection 1A, so as to be generally S-shaped when viewed from a side, the second substrate 7 at the top and the first substrate 6 at the bottom.
The second substrate 7 is disposed in the housing 22 at an upstream side in the puncturing direction so as to vertically face the two upper-side openings 24, and is fixed to a frame member (not shown). The terminals 12 are exposed from the upper-side opening 24 at an upstream side in the puncturing direction, and the electrodes 11 are exposed from the upper-side opening 24 at a downstream side in the puncturing direction.

The first substrate 6 is disposed in the housing 22 at a downstream side in the puncturing direction so as to face the front-side opening 23 in the longitudinal direction, and the puncture needle 9 is exposed from the front-side opening 23. The first substrate 6 is supported by a sliding member (not shown) provided in the housing 22. The sliding member slides along the longitudinal direction by a known mechanism.
By such sliding, the first substrate 6 slides along the longitudinal direction, through the accommodated position (ref: FIG. 10 (b)) at which the puncture needle 9 is disposed in the housing 22; and the puncturing position (ref: FIG. 10 (c)) at which the puncture needle 9 is disposed outside the housing 22.

When the glucose sensor 21D is not in use, the first substrate 6 is disposed at the accommodated position, as shown in FIG. 10 (b).
When in use, the first substrate 6 slides toward a downstream side in the puncturing direction, as shown in FIG. 10 (c), to be disposed at the puncturing position, and allows a patient to puncture, for example, his/her finger with the puncture needle 6 to cause a trace amount of bleeding.

Afterwards, the first substrate 6 is disposed again at the accommodated position, as shown in FIG. 10 (d), to be accommodated in the housing 22. Then, with the electrodes 11 being exposed from the upper-side opening 23, the patient himself/herself brings the punctured portion into contact with the electrodes 11. Then, the chemical agent 16 at the surface of the electrodes 11 is allowed to react with the blood, and the resistance value when a voltage is applied to the electrodes 11 changes according to the blood-sugar level in the blood.

Afterwards, in the same manner as above, external terminals 25 of a blood-sugar-level measuring device (not shown) are connected, and a glucose level is measured based on the changes in the resistance value. The measured glucose level is displayed at the display as the blood-sugar level.
Furthermore, in the glucose sensor 21D, the puncture needle 9 is accommodated in the housing 22, and therefore when a patient brings the punctured portion into contact with the electrodes 11, contact between the patient and the puncture needle 9 can be prevented, achieving an improvement in safety.

Although a blood-sugar-level measuring device including a CPU and a display is connected to the glucose sensors 21A to 21D in the above description, it is also possible to equip the glucose sensors 21A to 21D with a CPU and a display, and connect the terminals 12 and the external terminals 25 in the housing 22.
Furthermore, although the insulating base layer 3 is extended between the first substrate 6 and the second substrate 7 in the circuit boards for blood collection 1A to 1D in the description above, a metal reinforcing plate can be provided at a middle portion of the insulating base layer 3 between the first substrate 6 and the second substrate 7, according to purpose and use. In such a case, it is preferable that, at the reinforcing portion where the metal reinforcing plate is provided, flexibility is ensured by forming a mesh opening, or providing a plurality of slits along the width direction in the insulating base layer 3 and/or metal substrate, and further, forming the insulating base layer 3 thinner than other portions, or forming the metal substrate thinner than the first substrate 6 and the second substrate 7.

Although the circuit board for body fluid collection and biosensor of the present invention are illustrated as the circuit boards for blood collection 1A to 1D and the glucose sensors 21A to 21D in the above description, as long as it is a liquid in a living body, other than blood, without particular limitation, for example, an intracellular fluid or an extracellular fluid can be measured as a target object in the circuit board for body fluid collection and biosensor of the present invention. Examples of the extracellular fluid that can be listed include a blood plasma, an intercellular fluid, a lymph fluid, moistures in dense connective tissue, bone, sand cartilage, and a transcellular fluid, apart from blood mentioned above.

While the illustrative embodiments of the present invention are provided in the above description, such is for illustrative purpose only and it is not to be construed as limiting the scope of the present invention. Modification and variation of the present invention which will be obvious to those skilled in the art is to be covered by the following claims.

### Industrial Applicability

A circuit board for body fluid collection and a biosensor of the present invention are suitably used, for example, in the field where a blood-sugar level in blood is measured, and the like.

## Claims

1. A circuit board for body fluid collection comprising:
a first substrate including a puncture needle,
a second substrate that is disposed at an upstream side of the first substrate in a direction of puncturing with the puncture needle with a space provided therebetween,
a flexible insulation layer that extends between the first substrate and the second substrate, and
a conductive pattern provided on the flexible insulation layer, the conductive pattern integrally including an electrode, a terminal, and a wiring that electrically connects the electrode and the terminal.

2. The circuit board for body fluid collection according to Claim 1, wherein the terminal is disposed so as to overlap with the second substrate when projected in the thickness direction.

3. The circuit board for body fluid collection according to Claim 1, wherein the electrode is disposed so as to overlap with the first substrate when projected in the thickness direction.

4. The circuit board for body fluid collection according to Claim 1, wherein the electrode is disposed so as to overlap with a portion between the first substrate and the second substrate when projected in the thickness direction.

5. The circuit board for body fluid collection according to Claim 1, wherein the electrode is disposed so as to overlap with the second substrate when projected in the thickness direction.

6. A biosensor comprising a housing and the circuit board for body fluid collection according to Claim 1,
wherein the circuit board for body fluid collection is accommodated in the housing, the second substrate is fixed, and the first substrate is supported so as to be capable of advancing from inside the housing to outside the housing.
